(19) Europäisches Patentamt
European Patent Office
Office européen des brevets

(11) Publication number: 0 210 795 B1

# (12) EUROPEAN PATENT SPECIFICATION

(45) Date of publication of patent specification: 02.05.90

(51) Int. Cl.[5]: C 07 C 29/136, C 07 C 31/08, B 01 J 23/76

(21) Application number: 86305527.3

(22) Date of filing: 17.07.86

## (54) Hydrogenation of carboxylic acid esters to alcohols.

(30) Priority: 23.07.85 GB 8518576

(43) Date of publication of application: 04.02.87 Bulletin 87/06

(45) Publication of the grant of the patent: 02.05.90 Bulletin 90/18

(84) Designated Contracting States: AT BE DE FR GB IT LU NL SE

(56) References cited:
EP-A-0 123 517
EP-A-0 171 178
DD-A- 37 492
DE-A-3 443 277
US-A-4 405 819

Soviet Inventions Illustrated, section Ch; week C 03, February 27, 1980 Derwent Publications Ltd., London, D 23

(73) Proprietor: BP Chemicals Limited
Belgrave House 76 Buckingham Palace Road
London, SW1W 0SU (GB)

(72) Inventor: Williams, Peter Sefton c/o BP Chemicals Limited
Saltend Hedon
Hull HU12 8DS (GB)

(74) Representative: Fawcett, Richard Fennelly et al
BP INTERNATIONAL LIMITED Patents Division
Chertsey Road
Sunbury-on-Thames Middlesex, TW16 7LN (GB)

EP 0 210 795 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates in general to a catalytic process for the production of alcohols from carboxylic acid esters. In particular, the present invention relates to a process for the production of alcohols by hydrogenating carboxylic acid esters in the presence of a reductively activated solid copper-containing catalyst.

The catalytic hydrogenation of carboxylic acid esters to alcohols is described in, for example DE—A—3401896, DE—A—3443277, EP—A—95408, DE—A—3217429, USP 4,346,240, BE892958, EP—A—36939 and USP 4,405,819.

DE—A—3443277 discloses the hydrogenolysis of aliphatic esters in the presence of carbon dioxide to promote the activity of a reduced copper and zinc oxide catalyst.

BE 892958 discloses the hydrogenolysis of a carboxylic acid ester to an alcohol by contacting a vapour phase mixture of the ester and hydrogen with a catalyst comprising a reduced mixture of copper oxide and zinc oxide at 75 to 300°C under an absolute pressure of 0.1 to 100 kh/sq cm.

USP 4405819 discloses carboxylic acid ester hydrogenation catalysts which are metallic and metallic oxide compounds of transition elements in sub-group I such as copper, sub-group II such as zinc, sub-group IV such as chromium, sub-group VII such as manganese, and sub-group VIII such as iron, optionally supported on bentonite, Fuller's Earth, activated charcoal, alumina, and the like. Copper chromite is mentioned as preferred.

The remainder of the aforesaid patents disclose the use of copper-free hydrogenation catalysts, for example rhenium (DE 3401896A), a support, rhodium and tin, germanium and/or lead (EP—A—95408), rhenium and a noble metal (DE 3217429), supported Group VIII metal containing alkali metal component (US 4346240) and a Group VIII metal, alkali metal and radical anion on a carbon support (EP—A—36939).

We have now found that the hydrogenation of a carboxylic acid ester to the corresponding alcohol can be accomplished with a high productivity and selectivity to alcohol using a reductively activated catalyst containing an essential components copper and at least one of magnesium, a lanthanide metal or an actinide metal.

Accordingly the present invention provides a process for the production of an alcohol by the hydrogenation of a carboxylic acid ester at elevated temperature and atmospheric or elevated pressure in the presence of a reductively activated solid copper-containing catalyst characterised in that in addition to copper the catalyst contains at least one of lanthanide metal or an actinide metal.

Suitable catalysts for use in the process of the present invention before reductive activation may be represented by the formula:

$$Cu_aM^1M^2_bA_cO_x \quad (I)$$

wherein

$M^1$ is at least one of a lanthanide metal or an actinide metal,

$M^2$ is selected from Ca, Mo, Rh, Mn, Pt, Cr, Zn, Al, Ti, V, Ru, Re, Pd, Ag and Au,

A is an alkali metal,

a is from 0.1 to 4, preferably from 0.5 to 3, more preferably from 0.7 to 2,

b is from 0 to 1.0,

c is from 0 to 0.5, and

x is a number such that the valence requirements of the other elements for oxygen is satisfied.

For the purpose of the present invention, lanthanide metals are defined as metals having atomic numbers from 57 to 71 inclusive and actinide metals are defined as metals having an atomic number from 89 to 103. Although embraced within the scope of these definitions, many of the metals are unstable and would not be suitable for use in commercial operations.

A preferred catalyst for use in the process of the present invention has the formula before reductive activation:

$$Cu_aThA_cO_x \quad (II)$$

wherein

A, a, c and x have the meanings assigned in formula (I).

Another preferred catalyst for use in the process of the invention has the formula before reductive activation:—

$$Cu_aCeA_cO_x \quad (III)$$

wherein

A, a, c and x have the meanings assigned in formula (I).

The cerium may be present admixed with other actinide metals, for example lanthanum, normally present in certain commercially available forms of ceria.

Of the alkali metals, sodium and potassium are preferred, with potassium being more preferred.

Suitably the value of c in the formulae (I) to (III) is less than 0.15.

The catalysts of formulae (I) to (III) may be prepared by a variety of methods including impregnation, precipitation or gelation. A suitable method, for example, comprises impregnating the oxide of the metal $M^1$ with a compound of copper thermally decomposable to the oxide. Any suitable impregnation technique including the incipient wetness technique or the excess solution technique, both of which are well-known in the art, may be employed. The incipient wetness technique is so-called because it requires that the volume of impregnating solution be pre-determined so as to provide the minimum volume of impregnating solution necessary to just wet the entire surface of the support, with no excess liquid. The excess solution technique, as the name implies, requires an excess of the impregnating solution, the solvent being thereafter removed usually by evaporation. Using a metal $M^2$, this may be impregnated on to the oxide of the metal $M^1$ in a similar manner to that described hereinabove or the oxide of the metal $M^2$ may be physically mixed with the oxide of the metal $M^1$ and the copper impregnated thereon. The alkali metal, when present, may be impregnated on to the oxide of the metal $M^1$ at any stage in the preparation of the catalyst.

The impregnation solution may suitably be either an aqueous solution or a non-aqueous, organic solution, for example an alcoholic solution.

Any soluble copper salt may suitably be employed, for example the nitrate, chloride, acetate or acetylacetonate.

It is preferred to produce the catalyst by precipitation, either by coprecipitation of the metals copper and $M^1$, and optionally also $M^2$, in the form of insoluble thermally decomposable compounds thereof or by precipitation of an insoluble thermally decomposable compound of copper, and optionally $M^2$, in the presence of the oxide of the metal $M^1$.

A particularly preferred process for producing a catalyst for use in the process of the invention comprises the steps of:

(I) precipitating at a temperature in the range from 0 to 100°C the metals copper and $M^1$ (and optionally also $M^2$) in the form of insoluble thermally decomposable compounds thereof using a precipitant comprising a carbonate and/or bicarbonate of an alkali metal and/or ammonium,

(II) recovering the precipitate obtained in step (I), and

(III) thermally decomposing heat decomposable compounds comprised in the precipitate recovered in step (II), the maximum temperature utilised in the thermal decomposition being in the range from 300 to 700°C.

Step I of the aforesaid process may be accomplished in a variety of ways, some being better than others in terms of the activity of the final catalyst. Thus, an embodiment (A) comprises bringing together in solution at a temperature below 50°C soluble salts of the metals copper and $M^1$ and a precipitant comprising a carbonate and/or bicarbonate of an alkali metal and/or ammonium. Alternatively, an embodiment (B) comprises bringing together in a substantially copper-free solution a soluble $M^1$ salt with a precipitant so as to precipitate an $M^1$ compound and thereafter in the presence of the precipitated $M^1$ compound bringing together a solution of a soluble copper salt with a precipitant so as to precipitate an insoluble thermally decomposable compound, said precipitations being carried out at a temperature below 50°C and said precipitant comprising a carbonate and/or a bicarbonate of an alkali metal and/or ammonium. After precipitation of the $M^1$ compound, the solution of the soluble copper salt may suitably be added to the precipitation $M^1$ compound without any intervening treatment, such as for example filtration, prior to precipitating the copper compound. Alternatively, the precipitated $M^1$ compound may be separated, washed and re-dispersed prior to precipitating the copper compound. Many variants on the aforesaid embodiments (A) and (B) are possible, for example instead of adding the precipitant to the salts, the salts may be added to the precipitant.

Whilst any soluble salt of copper and $M^1$ may be employed, such as for example carboxylates, acetylacetones, naphthenates, chlorides and the like, it is preferred to use the nitrates.

It is preferred to use aqueous solutions of the salts, though aqueous alcoholic solutions for example may be employed if desired.

Of the alkali metals, sodium and potassium are suitable, principally for reasons of availability and cost. Examples of suitable precipitants are sodium carbonate, sodium bicarbonate, potassium carbonate, potassium bicarbonate, ammonium carbonate and ammonium bicarbonate. Using alkali metal salts as the precipitant, the resulting catalyst precursor composition will almost certainly contain alkali metal cations, the amount of which may be reduced by washing or may be increased by further addition of alkali metal cations. Compositions free from alkali metal [c in the formula (I) equal to zero] may suitably be produced using as the precipitant either ammonium carbonate or ammonium bicarbonate, even more preferably ammonium bicarbonate. Ammonium carbonate may suitably be used in a commercially available form, which comprises a mixture of ammonium bicarbonate and ammonium carbonate.

The amounts of the soluble copper and $M^1$ salts and precipitant employed should be such as to satisfy the stoichiometry of the formula (I).

In order to improve the homogeneity of the catalyst it is preferred to agitate the mixture during precipitation, suitably by mechanical stirring.

Addition of the precipitant in the preparation of ceria- and thoria-containing catalysts causes the initially low pH to rise. Generally, it will be found that precipitation of the cerium compound for example is

substantially complete at a pH of about 4. In the precipitation of the copper component of the catalysts the final value of the pH is suitably in the range from 5.0 to 7.0, preferably from 5.5 to 6.5, even more preferably from 5.9 to 6.3. The steps taken prior to achieving the final pH may vary considerably. Thus, the precipitant may be added until a pH in the aforesaid range is achieved, whereupon the addition of a further precipitant may be discontinued, thereby arresting the rise in the pH. Alternatively, the rise in pH may be allowed to continue by further addition of precipitant until it reaches a value in the range from 9 to 10 (at which point it will generally be found that precipitation is complete) and the pH of the mixture thereafter adjusted to a final value in the aforesaid range by addition of acid. In another alternative, the pH of the mixture may be maintained at a value in the aforesaid range by the addition of a suitable buffer.

It is further preferred to carry out step (I) in an atmosphere of carbon dioxide. Though we do not wish to be bound in any way by theory, it is believed that the presence of carbon dioxide may stabilise any carbonate phase and improve the dispersion of the metallic phases in the composition.

In step (II) of the process the precipitate obtained in step (I) is recovered. This may suitably be accomplished by filtration but other methods for separating solids from liquids, for example centrifugation, may be employed. After recovery, it is preferred to wash the precipitate, suitably with water, so as to remove unwanted residual soluble matter. Thereafter, the precipitate may be dried, suitably at an elevated temperature below 150°C, for example about 120°C.

Irrespective of the process employed, any thermally decomposable compounds of copper and, possibly also $M^1$, must be thermally decomposed in order to produce the composition of formula (I). The maximum temperature used during the thermal decomposition should not exceed a temperature in the range from 300 to 700°C, preferably from 400 to 600°C. The rate at which the temperature is increased may be varied over a wide range. It is preferred to maintain the thermally decomposable compounds at the maximum temperature for an extended period, for example from 1 to 200 hours. It is preferred to conduct the decomposition in a non-reducing atmosphere, suitably in a stream of either an inert gas, for example nitrogen, or an oxygen-containing gas, for example, air.

After decomposition the product comprises a metal oxide composition convertible by reductive activation into a catalyst for use in the process of the invention.

Reductive activation may suitably be effected, at least initially, with reducing gas substantially free from carbon monoxide, for example hydrogen preferably diluted with inert gas. A very suitable gas mixture is 5% hydrogen in nitrogen. Following initial reduction with a reducing gas other than carbon monoxide, subsequent reduction may be effected in carbon monoxide-containing gases, for example carbon monoxide/hydrogen mixtures. Suitably, reductive activation in this manner may be carried out as a discrete step prior to use as a catalyst, either 'in situ' or prior to loading into the reactor.

The pressure employed during the reductive activation step may suitably be in the range from 1 to 100 bar, the temperature may suitably be up to 300°C and periods of 24 hours or longer may be employed. Reductive activation may be effected at two or more temperatures, for example a period at 150°C followed by a further period at 200°C.

Suitable carboxylic acid esters for use in the process of the present invention are esters of $C_1$ to $C_{24}$ acyclic mono- or di-, saturated or unsaturated, straight — or branched — chain carboxylic acids. Suitably the esters are alkyl esters, preferably lower alkyl esters. Examples of the carboxylic acid esters useful herein include but are not limited to methyl acetate, ethyl acetate, propyl acetate, allyl acetate, dimethyl maleate, dimethyl succinate, methyl methacrylate and methyl oxalate. Hydrogenation of the unsaturated function of unsaturated carboxylic acid esters may occur.

The product of the process is the corresponding alcohol, for example from ethyl acetate there is obtained ethanol. Different products may be formed however where the initial reaction product can undergo other reactions, for example self-condensation. Thus, for example, gamma-butyrolactone may be formed from dialkyl esters of succinic acid.

The hydrogen used herein may be provided from any conventional source and may contain impurities, such as carbon monoxide and/or nitrogen for example.

Whilst there may be tolerated in the reactants the amounts of water normally associated with commercially available products, it is preferred to avoid larger amounts of water which might otherwise cause hydrolysis of the ester reactant.

The process may be operated either batchwise or continuously, preferably continuously, and either in the liquid phase or the vapour phase. In general, it is preferred to hydrogenate higher carbon number esters in the liquid phase and lower carbon number esters in the vapour phase. For vapour phase operation, using lower carbon number esters, the ratio of hydrogen to organic vapour feed is preferably in the range from 1:1 to 10:1 (on a molar basis). For continuous operation the organic feed rate is suitably in the range from 0.1 to 10 kg feed/kg catalyst/hour.

For liquid phase operation a solvent inert under the reaction conditions may be employed.

The process is preferably operated at an elevated temperature in the range from 150 to 300°C and an elevated pressure in the range from 10 to 100 barg.

The process of the present invention will now be further illustrated by reference to the following Examples.

A. Catalyst Preparation

Catalyst A

3.8 g of $Cu(NO_3)_2 2.5H_2O$ was dissolved in 25 ml deionised water then added to 5 g $CeO_2$ (commercially obtained, BET surface area 13 $m^2/g$) wetted with 10 ml of deionised water. After standing for 17 hours the water was removed on a rotary evaporator and the recovered solid dried overnight at 110°C in vacuo. The dried material was then calcined in air at 550°C for 17 hours at Gas Hourly Space Velocity (GHSV) = 2000. After cooling and purging with nitrogen, the catalyst was heated under hydrogen to 220°C over a period of 2 hours, and then held at the final temperature for a period of 2 hours. The catalyst was then cooled under hydrogen prior to passivation using nitrogen containing trace amounts of oxygen. The sample was subsequently stored under moisture free conditions.

Catalyst B

A copper/thoria catalyst was prepared by the coprecipitation of a 1:1 molar mixture of copper nitrate and thorium nitrate in aqueous solution at room temperature using ammonium bicarbonate as the precipitant. The final pH was 7. The precipitate was collected and dried in vacuo. It was then calcined in a stream of flowing nitrogen by heating to 450°C at a rate of 1°/min and then holding at 450°C for 6 hours. It was then cooled under a stream of nitrogen. The copper content was about 25% by weight.

B. Catalyst Testing

(i) Hydrogenation of Ethyl Acetate

Example 1

Ethyl acetate and 0.5 g of Catalyst A were charged to a 300 ml stainless steel autoclave equipped with a stirrer. The autoclave was pressurised with hydrogen to a pressure of 60 barg, heated at 230°C for 12 hours, then cooled and the liquid and gaseous products analysed by gas liquid chromatography (glc).

The results are collected in Table 1.

TABLE 1

| Example | Catalyst | Ethyl Acetate Charge (g) | Initial Pressure (RT) (barg) | Ethanol Productivity (g/gcat/h) | Selectivity (%) |
|---|---|---|---|---|---|
| 1 | A | 89.5 | 60 | 0.54 | 99 |

Example 2

1.26 g of Catalyst B was loaded into a stainless steel reactor having an internal diameter of about 6 to 7 mm. The reactor was placed in a furnace under a flowing atmosphere of $CO/H_2$ in a ratio from 1:2. The flow rate of the gas was 60 ml/min and the pressure was 100 psig. The reactor was heated to 200°C for 3 hours, then heated to 220°C for 7 hours.

Ethyl acetate vapour was then fed into the reactor by pumping liquid ethyl acetate into a preheat zone in the gas stream. The ratio of ester to hydrogen was about 1:8.5 and the pressure was maintained to 6.7 barg. The exit gas stream was analysed by gas chromatography. The results are shown in Table 2.

TABLE 2

| Time on Stream[1] (hrs) | P/Barg | Temperature °C | Molar Ratio $H_2$:EtOAc | GHSV (STP) | Relative Weights Normalised to 100% | | | |
|---|---|---|---|---|---|---|---|---|
| | | | | | EtOAc | EtOH | $Me_2CHOH$ | $C_4$[2] |
| 5.0 | 6.67 | 202 | 8.5:1 | 2200 | 15 | 78 | 3.1 | ca 3 |
| 9.0 | 10 | 202 | 4.2:1 | 2500 | 34 | 58 | 2.2 | ca 2 |
| 16.0 | 10 | 220 | 4.2:1 | 2500 | 16 | 68 | 7.2 | ca 7 |
| 25.0 | 10 | 220 | 4.2:1 | 2500 | 16 | 68 | 7.2 | ca 7 |

1 From commencement of ethyl acetate feed.
2 Trace amounts of acetic acid, methane and ethane also formed.

**Claims**

1. A process for the production of an alcohol by the hydrogenation of a carboxylic acid ester at elevated temperature and atmospheric or elevated pressure in the presence of a reductively activated solid copper-containing catalyst characterised in that in addition to copper the catalyst contains at least one of a lanthanide metal or an actinide metal.

2. A process according to claim 1 wherein the catalyst before reductive activation is represented by the formula:—

$$Cu_aM^1M^2{}_bA_cO_x \qquad (I)$$

wherein

$M^1$ is at least one of a lanthanide metal or an actinide metal,

$M^2$ is selected from Ca, Mo, Rh, Mn, Pt, Cr, Zn, Al, Ti, V, Ru, Re, Pd, Ag and Au,

A is an alkali metal,

a is from 0.1 to 4,

b is from 0 to 1.0,

c is from 0 to 0.5, and

x is a number such that the valence requirements of the other elements for oxygen is satisfied.

3. A process according to claim 1 wherein the catalyst before reductive activation has the formula:—

$$Cu_aThA_cO_x \qquad (II)$$

wherein

A, a, c and x have the meanings assigned in formula (I).

4. A process according to claim 1 wherein the catalyst before reductive activation has the formula:—

$$Cu_aCeA_cO_x \qquad (III)$$

wherein

A, a, e and x have the meanings assigned in formula (I).

5. A process according to any one of claims 2 to 4 wherein the value of a in the formulae (I) to (III) is in the range from 0.5 to 3.

6. A process according to any one of claims 2 to 5 wherein the alkali metal A is either sodium or potassium.

7. A process according to any one of claims 2 to 6 wherein the value of c in the formulae (I) to (III) is less than 0.15.

8. A process according to any one of the preceding claims wherein the catalyst is supported.

9. A process according to any one of claims 2 to 7 wherein the catalyst of formulae (I) to (III) is produced by a process comprising the steps of:

(I) precipitating at a temperature in the range from 0 to 100·C the metals copper and $M^1$ (and optionally also $M^2$) in the form of insoluble thermally decomposable compounds thereof using a precipitant comprising a carbonate and/or bicarbonate of an alkali metal and/or ammonium,

(II) recovering the precipitate obtained in step (I), and

(III) thermally decomposing heat decomposable compounds comprised in the precipitate recovered in step (II), the maximum temperature utilised in the thermal decomposition being in the range from 300 to 700°C.

10. A process according to any one of the preceding claims wherein the catalyst is reductively activated prior to use in the process.

11. A process according to claim 10 wherein reductive activation is effected, at least initially, with reducing gas substantially free from carbon monoxide.

12. A process according to any one of the preceding claims wherein the carboxylic acid ester is ethyl acetate and the alcohol produced is ethanol.

**Patentsprüche**

1. Verfahren zur Herstellung eines Alkohols durch Hydrierung eines Carbonsäureesters bei erhöhter Temperatur und bei atmosphärischem oder erhöhtem Druck in Anwesenheit eines reduktive aktivierten festen kupferhaltigen Katalysators, dadurch gekennzeichnet, daß der Katalysator zusätzlich zu Kupfer wenigstens ein Lanthanidenmetall oder ein Actinidenmetall enthält.

2. Verfahren nach Anspruch 1, worin der Katalysator vor der reduktiven Aktivierung der Formel

$$CU_aM^1M^2{}_bA_cO_x \qquad (I)$$

entspricht, worin

$M_1$ wenigstens ein Lanthanidenmetall oder ein Actinidenmetall bedeutet,

$M^2$ unter Ca, Mo, Rh, Mn, Pt, Cr, Zn, Al, Ti, V, Ru, Re, Pd, Ag und Au ausgewählt ist,
A ein Alkalimetall bedeutet,
a einen Wert von 0,1 bis 4 aufweist,
b einen Wert von 0 bis 1,0 aufweist,
c einen Wert von 0 bis 0,5 aufweist und
x eine solche Zahl ist, daß den Valenzerfordernissen der anderen Elemente für Sauerstoff entsprochen wird.

3. Verfahren nach Anspruch 1, worin der Katalysator vor der reduktiven Aktivierung der Formel

$$Cu_aThA_cO_x \quad (II)$$

entspricht, worin
A, a, c und x die in Formel (I) angegebenen Bedeutungen aufweisen.

4. Verfahren nach Anspruch 1, worin der Katalysator vor der reduktiven Aktivierung der Formel

$$Cu_aCeA_cO_x \quad (III)$$

entspricht, worin
A, a, c und x die in Formel (I) angebegenen Bedeutungen aufweisen.

5. Verfahren nach einem der Ansprüche 2 bis 4, worin der Wert von a in den Formeln (I) bis (III) im Bereich von 0,5 bis 3 liegt.

6. Verfahren nach einem der Ansprüche 2 bis 5, worin das Alkalimetall entweder Natrium oder Kalium ist.

7. Verfahren nach einem der Ansprüche 2 bis 6, worin der Wert von c in den Formeln (I) bis (III) unter 0,15 liegt.

8. Verfahren nach einem der vorstehenden Ansprüche, worin der Katalysator ein Trägerkatalysator ist.

9. Verfahren nach einem der Ansprüche 2 bis 7, worin der Katalysator der Formeln (I) bis (III) nach einem Verfahren hergestellt wird, daß die folgenden Stufen umfaßt:
(I) Ausfällen der Metalle Kupfer und $M^1$ (und gegebenenfalls auch $M^2$) bei einer Temperatur im Bereich von 0 bis 100°C in Form von unlöslichen, thermisch zersetzbaren Metallverbindungen unter Verwendung eines Fällungsmittels, das ein Carbonat und/oder Bicarbonat eines Alkalimetalles und/oder von Ammonium umfaßt,
(II) Gewinnen des in Stufe (I) gewonnenen Niederschlages und
(III) thermisches Zersetzen von in dem in Stufe (II) gewonnenen Ausfällungsprodukt enthaltenen wärmezersetzbaren Verbindungen, wobei die bei der thermischen Zersetzung angewendete Maximaltemperatur im Bereich von 300 bis 700°C liegt.

10. Verfahren nach einem der vorstehenden Ansprüche, worin der Katalysator vor seiner Verwendung in dem Verfahren reduktiv aktiviert wird.

11. Verfahren nach Anspruch 10, worin die reduktive Aktivierung, zumindest zu Beginn, mit einem im wesentlichen von Kohlenmonoxid freien reduzierenden Gas ausgeführt wird.

12. Verfahren nach einem der vorstehenden Ansprüche, worin der Carbonsäureethylester Ethylacetat ist und der gebildete Alkohol Ethanol ist.

**Revendications**

1. Procédé de production d'un alcool par l'hydrogénation d'un ester d'acide carboxylique à température élevée et sous la pression atmosphérique ou sous pression élevée, en présence d'un catalyseur contenant du cuivre solide activé par voie réductrice, procédé caractérisé en ce qu'en plus du cuivre, le catalyseur contient au moins un métal qui est un métal de la famille des lanthanides ou un métal de la famille des antinides.

2. Procéde selon la revendiction 1, dans lequel le catalyseur est, avant son activation réductrice, représenté par la formule I

$$CU_aM^1M^2_bA_cO_x \quad (I)$$

dans laquelle $M^1$ représente au moins un métal de la famille des lanthanides ou un métal de la famille des actinides,
$M^2$ est choisi parmi Ca, Mo, Rh, Mn, Pt, Cr, Zn, Al, Ti, V, Ru, Re, Pd, Ag et Au,
A représente un métal alcalin,
a vaut de 0,1 à 4,
b vaut de 0 à 1,0,
c vaut de 0 à 0,5 et
x est un nombre tel que les exigences de valence des autres élémentes par rapport à l'oxygène sont satisfaites.

3. Procédé selon la revendication 1, dans lequel avant l'activation réductrice, le catalyseur a pour formule

$$Cu_aThA_cO_x \quad (II)$$

dans laquelle
A, a, c et x ont les sens indiqués à propos de la formule (I).

4. Procédé selon la revendication 1, dans lequel, avant l'activation réductrice, le catalyseur a pour formule

$$Cu_aCeA_cO_x \quad (III)$$

dans laquelle
A, a, c et x ont les sens attribués dans le cas de la formule (I).

5. Procédé selon l'une quelconque des revendications 2 à 4, dans lequel la valeur de a, dans les formules (I) à (III), se situe entre 0,5 et 3.

6. Procéde selon l'une quelconque des revendications 2 à 5, dans lequel le métal alcalin A est du sodium ou du potassium.

7. Procédé selon l'une quelconque des revendications 2 à 6, dans lequel la valeur de c, dans les formules (I) à (III), est inférieure à 0,15.

8. Procédé selon l'une quelconque des revendications précédentes, dans lequel le catalyseur est sur un support ("catalyseur supporté").

9. Procédé selon l'une quelconque des revendications 2 à 7, dans lequel le catalyseur répondant aux formules (I) à (III) est produit par un procédé comprenant les étapes consistant

(I) à précipiter, à une température comprise entre 0 et 100°C, les métaux cuivre et $M^1$ (et éventuellement aussi $M^2$) sous la forme de leurs composés insolubles, décomposables thermiquement, à l'aide d'un agent de précipitation comprenant un carbonate et/ou un bicarbonate d'un métal alcalin et/ou d'ammonium,

(II) à récupérer le précipité obtenu dans l'étape (I), et

(III) à décomposer par voie thermique les composés décomposables par la chaleur et qui sont compris dans le précipité récupéré à l'étape (II), la température maximale utilisée lors de la décomposition thermique se situant entre 300 et 700°C.

10. Procédé selon l'une quelconque des revendications précédentes, dans lequel le catalyseur est activé par réduction, avant de servir dans le procédé.

11. Procédé selon la revendication 10, dans lequel l'activation réductrice est effectuée, au moins initialement, à l'aide d'un gaz réducteur quasi-totalement dépourvu de monoxyde de carbone.

12. Procédé selon l'une quelconque des revendications précédentes, dans lequel l'ester d'acide carboxylique est l'acétate d'éthyle, et l'alcool produit est l'éthanol.